# FASCICULE DE BREVET EUROPEEN

(11) **EP 2 984 132 B1**
(45) Date de publication et mention de la délivrance du brevet: **30.11.2022**
(21) Numéro de dépôt: 14715638.4
(22) Date de dépôt: 08.04.2014
(51) Int. Cl.: C08K 5/10, C08K 5/101, C08K 5/11, C08K 5/12

(54) **COMPOSITION PLASTIFIANTE**
PLASTIFIZIERUNGSZUSAMMENSETZUNG
PLASTICIZING COMPOSITION

(30) Priorité: 10.04.2013 FR 1353227; 19.06.2013 EP 13172646
(43) Date de publication de la demande: 17.02.2016
(73) Titulaire: BASF SE, 67056 Ludwigshafen am Rhein (DE)
(72) Inventeur: RHERS, Bouchra, F-69003 Lyon (FR); MARTINZ, Daniel, B-1200 Bruxelles (BE)
(74) Mandataire: BASF IP Association
(86) Numéro de dépôt international: PCT/EP2014/057071
(87) Numéro de publication internationale: WO 2014/166955

(56) Documents cités:
- EP-A1- 1 454 958
- EP-A1- 1 491 523
- DE-A1- 19 938 159
- FR-A1- 2 400 058
- FR-A1- 2 898 356
- FR-A1- 2 898 356

## Description

L'invention concerne une composition plastifiante comprenant une résine de type chlorure de vinyle et un agent plastifiant. La présente invention concerne aussi les utilisations de la composition plastifiante.

### Etat de la technique

Les agents plastifiants majoritairement utilisés sont communément appelés "General Purpose" (GP) phtalates, et représentent ainsi 88% de la consommation globale (6.7 MTon in 2011)1. Dans la famille GP phtalates, on trouve le DEHP (di-2-ethylhexyl phtalate), le DINP (di-isononylphtalate) et le DIDP (di-isodecylphtalate). La somme de ces trois phtalates représente de l'ordre 83% de la demande mondiale de plastifiants. Le DEHP à lui seul représente environ 50% de la demande mondiale.

### But de l'invention

L'invention a pour but général de fournir de nouvelles compositions plastifiantes.

L'agent plastifiant selon l'invention présente une bonne performance pour des formulations de types différents (mousse, film et pâte). Ce plastifiant gélifie rapidement, a une viscosité très basse et vieilli très bien. Le débullage efficace est l'une de ces caractéristiques phares. Les mousses préparées avec ce plastifiant ont des densités basses, une grande vitesse d'expansion et d'excellentes qualités.

Un procédé permettant l'obtention de l'agent plastifiant est également divulgué.

Les inventeurs ont mis au point un procédé de préparation de composés diesters à partir de dinitriles et/ou diacides mettant en œuvre une réaction d'hydrolyse suivie d'une étape d'estérification. Une étape de décoloration et/ ou de purification permet l'obtention d'un diester pur.

La divulgation concerne encore plus particulièrement un procédé de préparation de composés diesters ramifiés tels que le diester d'acide 2-méthylglutarique (aussi appelé MGA) et le diester d'acide 2-éthylsuccinique (aussi appelé ESA).

Le diester à base de MGA seul ou en mélange avec le diester à base mélange de d'ESA possède un avenir prometteur dans le milieu des industries chimiques. Il s'agit d'un plastifiant ayant des propriétés plastifiant pouvant remplacer les plastifiants à base de phtalates et sans phtalates. Il peut être utilisé comme substitut de plastifiant tout seul ou en mélange avec d'autres plastifiants, pour des préparations/formulations de polyamides, de béton, de polyesters, de polyuréthanes, des résines chlorure de vinyls, des co-polymères ethylene-vinyl d'acétate, de caoutchouc, de mastics ou de leurs mélanges.

Le diester est obtenu soit à partir de dinitriles en passant par un intermédiaire diacide, soit à partir de diacide directement.

WO 2007/141404 propose de préparer des composés diacides par hydrolyse de composés dinitriles en présence d'un excès de composés basiques hydroxyle, le sel carboxylate obtenu étant ensuite mis à réagir avec un acide minéral pour récupérer le composé diacide.

WO 2008/062058 propose de préparer des composés diacides par hydrolyse de composés dinitriles en présence d'un excès d'acide fort minéral.

WO2007/101929 propose la préparation de diester par hydrolyse de dinitriles par voie acide ou par voie basique, l'intermédiaire diacide est ensuite estérifié en présence de méthanol afin d'obtenir le diester méthylique correspondant.

FR 2400058 décrit une composition de type PVC avec un plastifiant. Le document mentionne comme plastifiants de phtalates aliphatiques comme des phtalates de dialkyle, des adipates de dialkyle, par example des adipates de diisodécyle, et des sébaçates de dialkyle, préférentiellement les phtalates d'alkyle. Dans les examples, les phtalates de diisodécyle et de diisooctyle sont utilisées.

La présente invention concerne une résine de type chlorure de vinyle et un agent plastifiant qui comporte au moins un diester de formule générale (I) :

RO-OC-A-CO-OR (I)

dans laquelle :
- A est une chaine aliphatique linéaire ou branchée en C4, et
- R est un alkyle, un cycloalkyle ou un aryle,
et que l'agent plastifiant est un mélange de :
- 70 à 95% en poids de diester d'acide méthylglutarique,
- 5 à 30% en poids diester d'acide éthylsuccinique, et
- 0 à 10% en poids diester d'acide adipique,
ou est un mélange de :
- 95 à 100% en poids de diester d'acide méthylglutarique, et
- 0 à 5 % en poids de diester d'acide éthylsuccinique.

De manière préférée R est le cyclohexyle ou le 2-éthyl-hexyle.

Selon un mode d'exécution de l'invention, l'agent plastifiant comprend un mélange comprenant :
de 70 à 95% en poids de diester d'acide methylglutarique
de 5 à 30% en poids diester d'acide ethylsuccinique
de 0 à 10% en poids diester d'acide adipique

Selon un autre mode d'exécution de l'invention, l'agent plastifiant comprend un mélange comprenant :
de 95 à 100% en poids de diester d'acide méthylglutarique, et
de 0 à 5 % en poids de diester d'acide éthylsuccinique.

Selon un mode préféré de réalisation de l'invention, le diester est le di-cyclohexyle de l'acide méthylglutarique (DCH-MGA).

Selon au autre mode de réalisation de l'invention, le diester est le di-éthyl-hexyle de l'acide adipique (DCH-AA).

Selon au troisième mode de réalisation de l'invention, le diester est le di-éthyl-hexyle de l'acide méthylglutarique (DEH-MGA).

Le plastifiant de cette invention peut être préparé par estérification de diacides avec ces alcools cycliques.

### Réactions :

Lors de la réaction d'hydrolyse du composé dinitrile en présence d'acide on a la formation de diacides et de sel hydrogénosulfate d'ammonium. Ce dernier peut être valorisé sous forme de sulfates d'ammonium par sa réaction avec une autre molécule d'ammoniaque.

Schématiquement, on peut représenter la réaction d'hydrolyse d'un composé dinitrile, par exemple le 2-méthylglutaronitrile, en composé intermédiaire diacide du procédé de l'invention de la manière suivante :

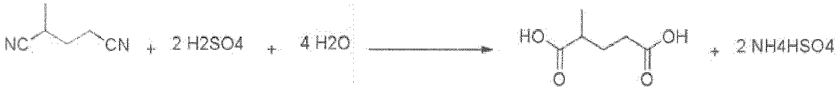

Dans le milieu réactionnel, le composé diacide est ensuite mis en réaction d'estérification avec un alcool, par exemple de cyclohexanol, pour donner un diester du procédé de la divulgation de la manière suivante :

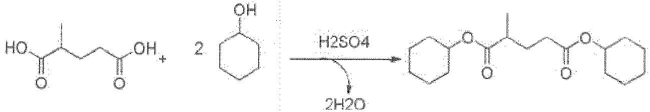

### Exemples

Les pourcentages sont donnés en poids par rapport au poids de la composition totale. Les températures sont données en degré Celsius (°C).

### Exemple 1

Procédure générale de synthèse de plastifiants par estérification.

Dans un réacteur double enveloppé de 1000ml équipé d'un système de purge en bas, d'un système d'agitation, d'une arrivée d'azote, d'un thermomètre, d'un Dean-Starck, d'une pompe pour assurer le vide et d'une ampoule équipée d'un système de refroidissement et de chauffe par laquelle sont ajoutés l'acide et d'alcool dont la stœchiométrie est 1 : 2. Le catalyseur, par exemple l'acide sulfurique est ajouté à 80°C. La réaction se déroule à 140°C avec élimination de l'eau au fur et à mesure de l'avancement de la réaction. Le stripping à l'azote favorise ainsi son élimination et sa récupération dans le Dean-starck. Une fois la quantité d'eau co-produite est totalement collectée, on monte la température au-delà de 140°C et on baisse la pression afin d'éliminer l'excès d'alcool. Le milieu réactionnel est ensuite lavé plusieurs fois avec des saumures et à la fin avec de l'eau déminéralisée. Le reste de catalyseur est neutralisé par le carbonate de sodium. Une étape de décoloration par passage sur charbon peut être faite en fonction de niveau de coloration du milieu. La phase organique contenant le diester est par la suite purifiée et décolorée par distillation. Le diester pur et incolore est ainsi obtenu avec des bons rendements.

RO-OC-A-CO-OR

**Tableau 1: sommaire des diesters préparés par réaction d'alcool et diacides.**

| Nomenclature | A | R | MM (g/mol) |
|---|---|---|---|
| Succinate de bis(2-ethylhhexyle) | -(CH₂)₂- | - 2-Ethylhexyl | 342 |
| Glutarate de bis(2-ethylhhexyle) | -(CH₂)₃- | - 2-Ethylhexyl | 356 |
| Adipate de dicyclohexyle | -(CH₂)₄- | - Cyclohexyl | 310 |
| 2-MethylGlutarate de bis(2-ethylhhexyle) | -CH₂-CH₂-CH(CH₃)- | - 2-Ethylhexyl | 370 |
| 2-MethylGlutarate de dicyclohexyle | -CH₂-CH₂-CH(CH₃)- | - Cyclohexyl | 310 |
| 2-EthylSuccinate de bis(2-ethylhhexyle) | -CH₂CH(CH₂CH₃)- | - 2-Ethylhexyl | 370 |
| 2-EthylSuccinate de dicyclohexyle | -CH₂-CH(CH₂CH₃)- | - Cyclohexyl | 310 |
| Plast Fast = mélange de 95%MethylGlutarate de dicyclohexyle et 5% 2-EthylSuccinate de dicyclohexyle | 95% : -CH₂-CH₂-CH(CH₃)- | - Cyclohexyl | 310 |
| | 5% : -CH₂-CH(CH₂CH₃)- | | |

### Synthèse de composés à base de PVC : Plastification

Le Tableau 2 résume l'ensemble des plastifiants utilisés pour les tests avec les résines PVC sous différents formulations.

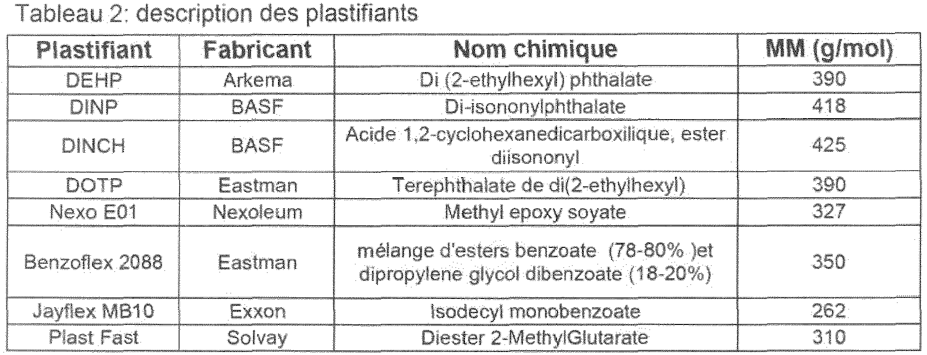

Un bilan comparatif des performances des plastifiants étudiés a été réalisé pour chaque formulation. Les critères de classement de performance des plastifiants en comparaison avec le DINP sont classés comme suit :

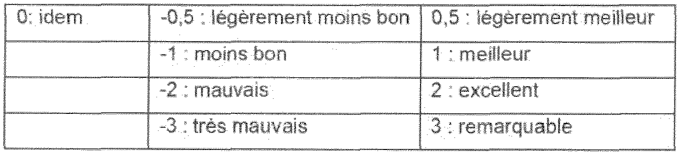

### Exemple 2

Chaque plastifiant énuméré au tableau 2 permet de réaliser des formulations transparentes selon le tableau 3 ci-dessous, à l'aide d'un mélangeur type mélangeur à vitesse moyenne à une température de 23°C.

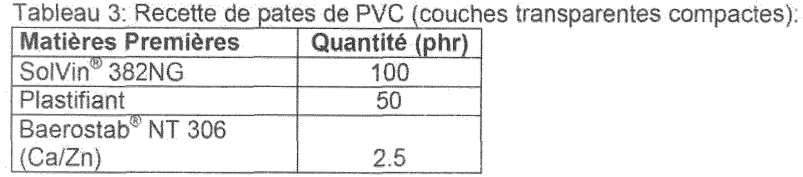

La comparaison des données obtenues pour les formulations synthétisées, à chaque fois en modifiant le plastifiant sont résumés dans le tableau 4.

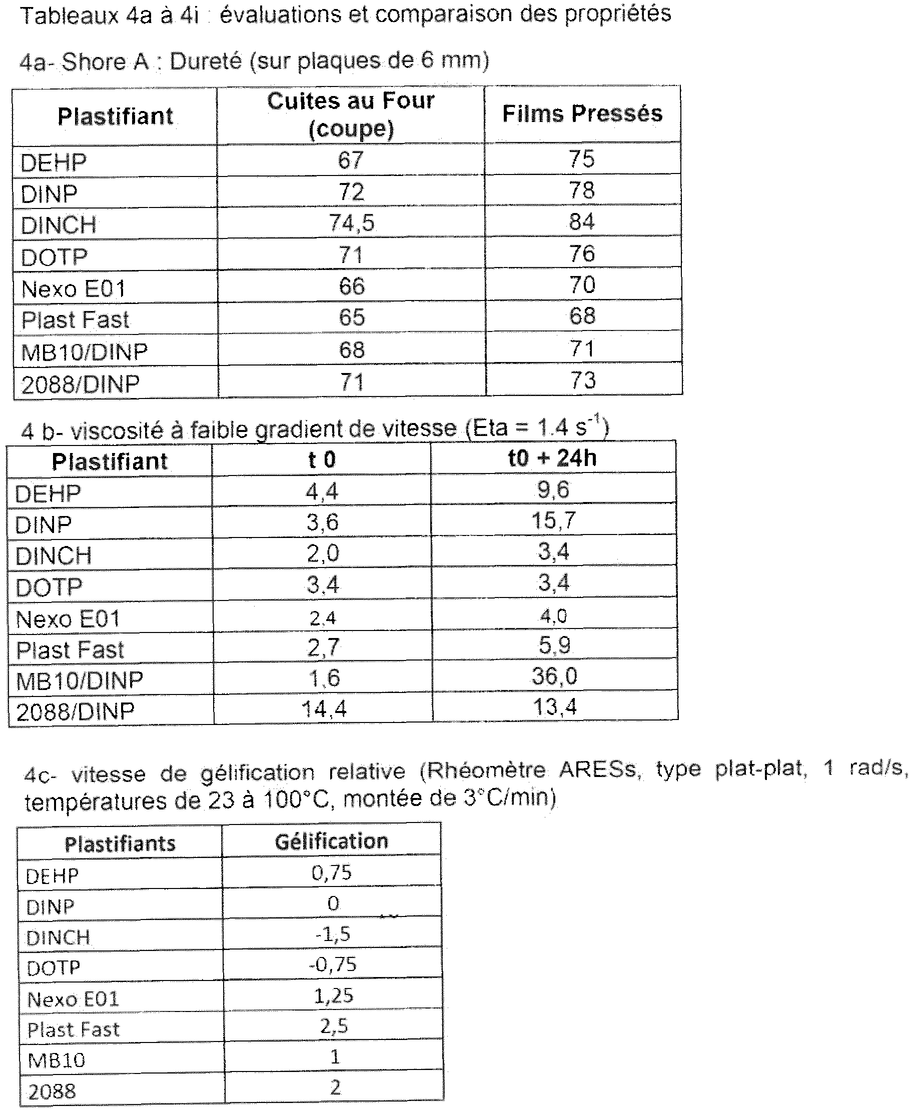

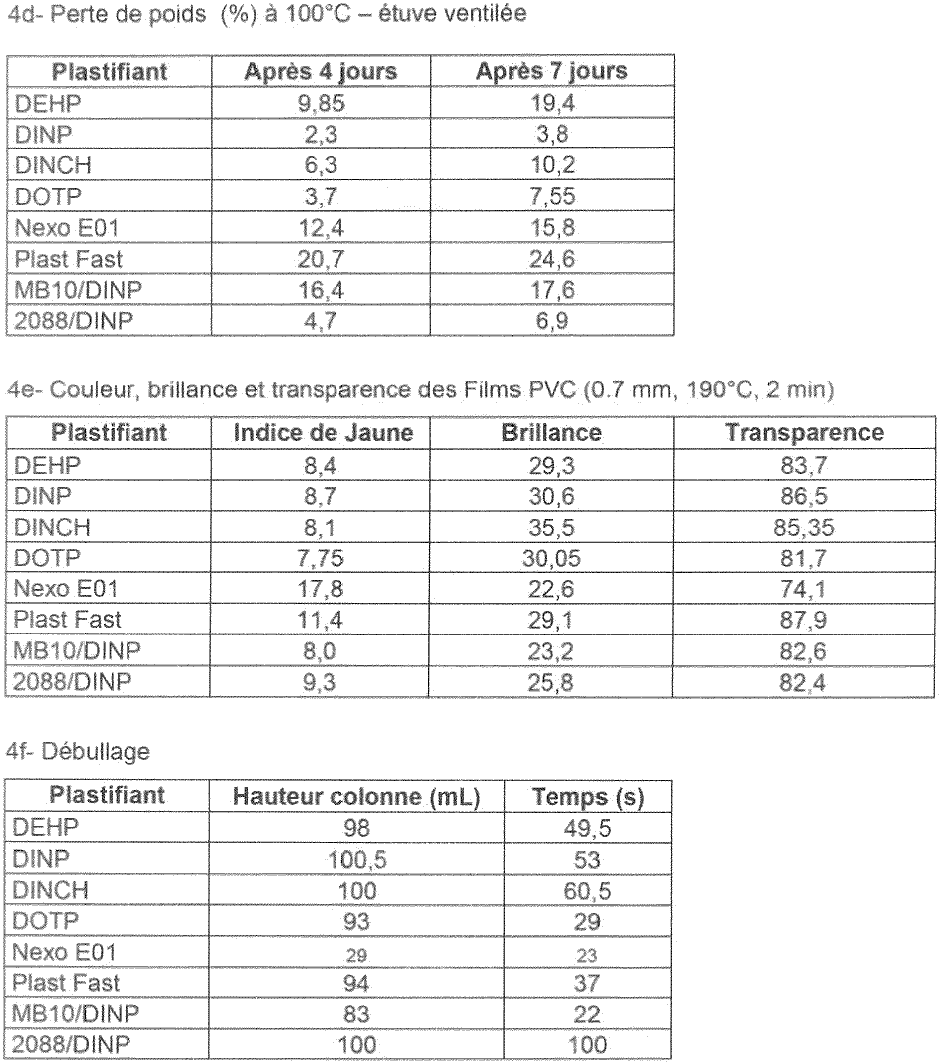

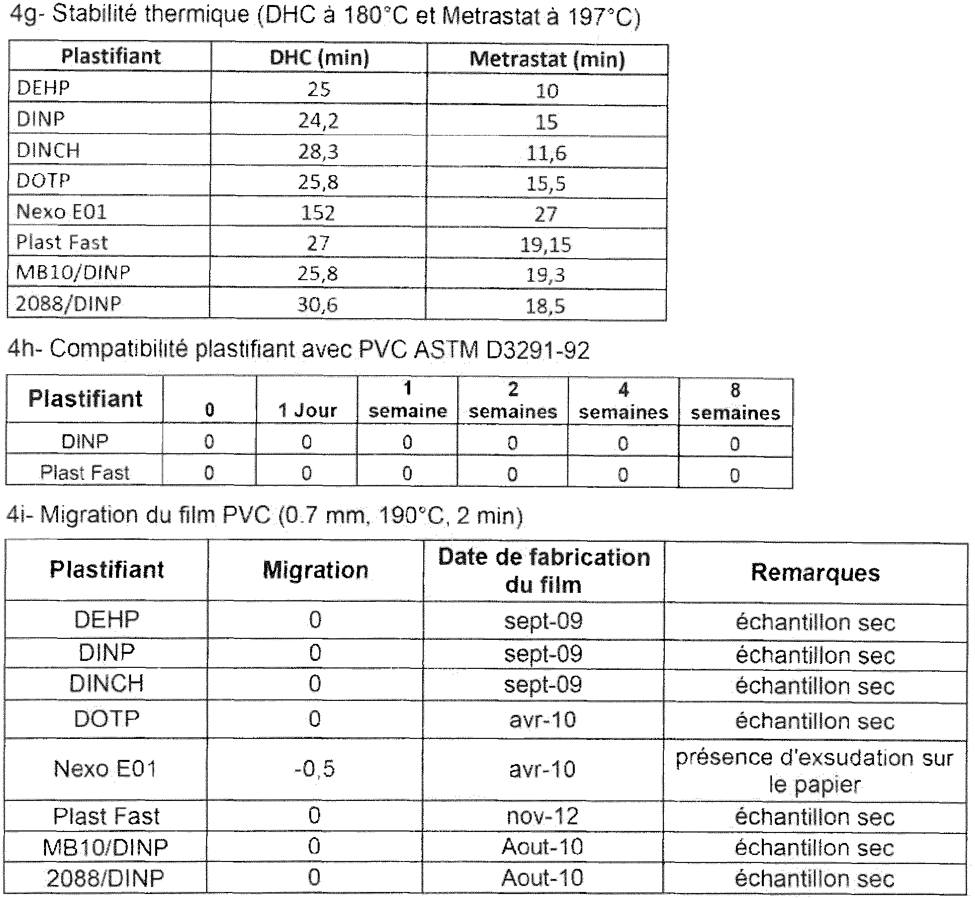

Les meilleures performances qui ressortent pour le plastifiant « Plast Fast » sont la gélification et le débullage.

Le tableau 5 récapitule le classement des plastifiants selon les notes attribuées.

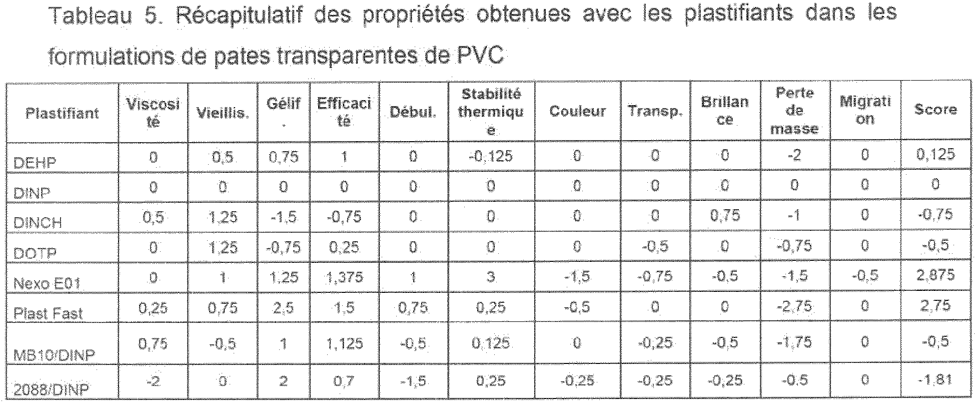

### Exemple 3

Des formulations mousses correspondant au tableau 6 ont été effectuées avec chaque plastifiant du tableau 2, dans un mélangeur de type à moyenne vitesse.

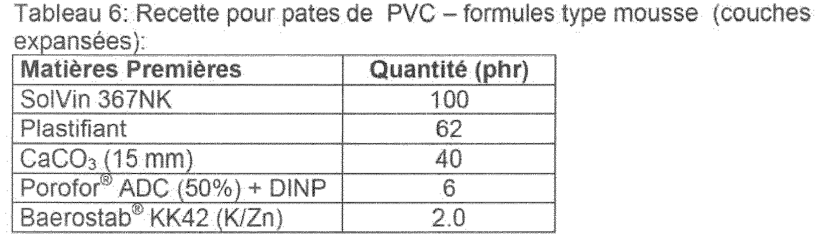

La comparaison des données obtenues pour les formulations synthétisées, à chaque fois en modifiant le plastifiant sont résumées dans le tableau 7.

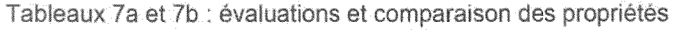

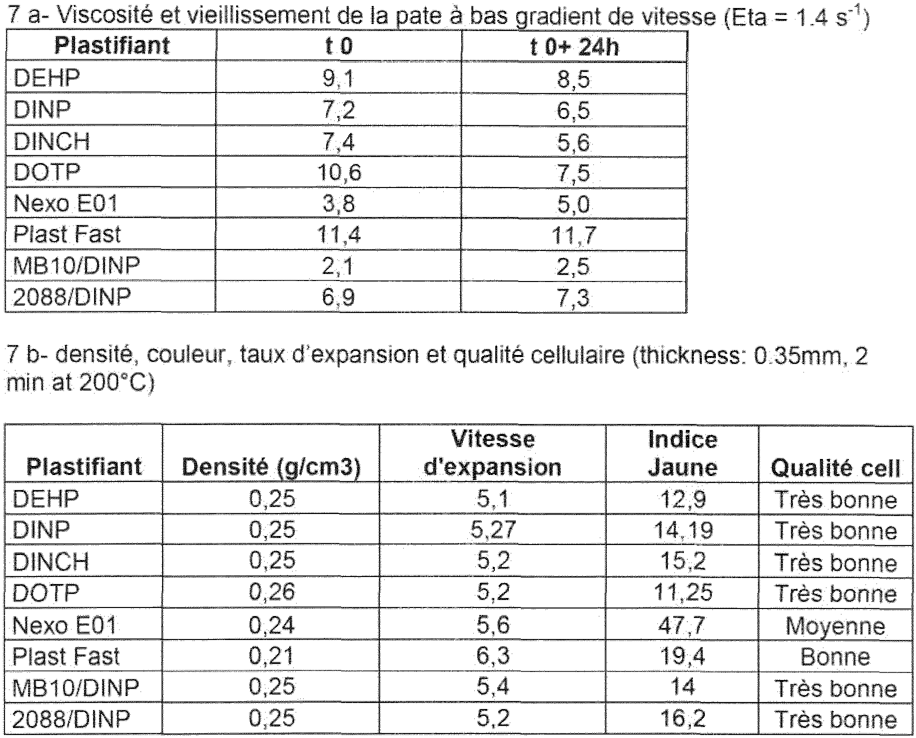

Le tableau 8 récapitule le classement des plastifiants selon les notes attribuées.

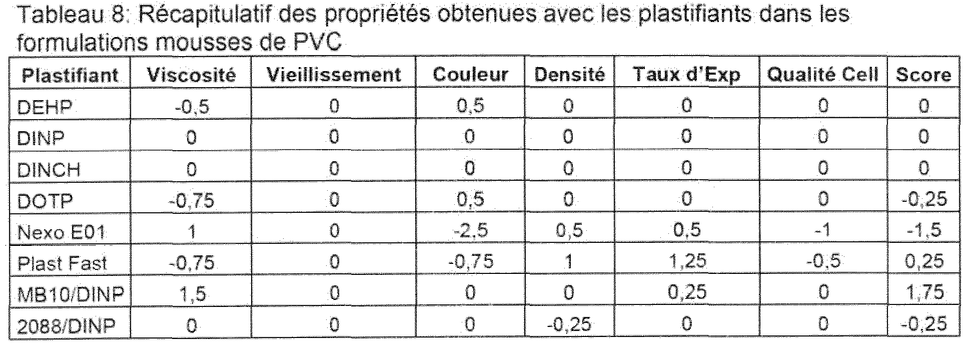

La comparaison générale de ces 2 formulations nous permet d'avoir le résultat de la performance globale des plastifiants. La moyenne du score des couches transparentes et le score des couches mousses nous donne le score global de performance pour chaque plastifiant et nous permet ainsi de les comparer. Le tableau 9 donne le score total des performances évaluées.

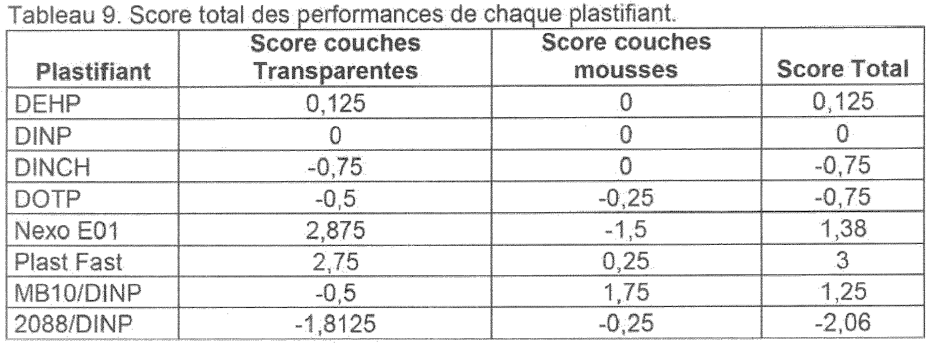

En moyenne, le « plast fast » présente une bonne performance pour les 2 recettes testées, il a le plus haut score en comparaison avec les autres plastifiants. Ce plastifiant gélifie rapidement, a une viscosité très basse et vieilli très bien. Le débullage efficace est l'une de ces caractéristiques phares. Les mousses préparées avec ce plastifiant ont des densités basses, une grande vitesse d'expansion et d'excellentes qualités.

## Revendications

1. Composition plastifiante comportant une résine de type chlorure de vinyle et un agent plastifiant qui comporte au moins un diester de formule générale (I) :
RO-OC-A-CO-OR (I)
dans laquelle :
- A est une chaine aliphatique linéaire ou branchée en C4, et
- R est un alkyle, un cycloalkyle ou un aryle,
et **caractérisée en ce que** l'agent plastifiant est un mélange de :
- 70 à 95% en poids de diester d'acide méthylglutarique,
- 5 à 30% en poids diester d'acide éthylsuccinique, et
- 0 à 10% en poids diester d'acide adipique,
ou est un mélange de :
- 95 à 100% en poids de diester d'acide méthylglutarique, et
- 0 à 5 % en poids de diester d'acide éthylsuccinique.

2. Composition selon la revendication 1, **caractérisée en ce que** R est un cycloalkyle substitué ou non, de 5 à 6 atomes de carbone, choisi parmi le cyclopentyle ou le cyclohexyle, ou un aryle choisi parmi le benzyle, phényle, et leurs isomères.

3. Composition selon la revendication 1, **caractérisée en ce que** R est un alkyl ramifié ou non, ayant de 4 à 10 d'atome de carbone, en particulier choisi parmi le n-butyle, t-butyle, n-pentyle, n-hexyle, n-heptyle, n-octyle, éthyl-hexyl, iso-octyle, n-nonyle, iso-nonyl, n-décyle, iso-décyle.

4. Composition selon l'une quelconque des revendications précédentes, **caractérisée en ce qu'**elle comprend en outre un ou plusieurs composés choisis parmi des stabilisants, les charges, des pigments, des biocides, du charbon noir, des promoteurs SV13/72235PCEP d'adhésion, des réducteurs de viscosité, des agents thixotropiques, des agents épaississants, des agents de gonflement, des dispersants, ou autres additifs.

5. Composition selon l'une quelconque des revendications précédentes, **caractérisée en ce que** l'agent plastifiant comprend en outre un ou plusieurs composés choisi parmi le phthalate, l'adipate, le benzoate, le triglycéride, ou autres polymères.

6. Utilisation de la composition selon l'une quelconque des revendications 1 à 5, pour préparer des articles sélectionnés parmi les jouets, films, feuilles, tubes flexibles, revêtements, câbles, dalles de sol, en particulier à base de vinyle, cuirs synthétiques, adhésifs et encres.

7. Utilisation de la composition selon l'une quelconque des revendications 1 à 5, pour la préparation d'articles par des procédés de mélange à sec ou extrusion.

8. Utilisation de la composition selon l'une quelconque des revendications 1 à 5, pour la préparation de plastisols de type pâte, film ou mousse.

## Patentansprüche

1. Weichmacherzusammensetzung, die ein Harz vom Vinylchlorid-Typ und einen Weichmacher umfasst, der mindestens einen Diester der allgemeinen Formel (I) umfasst:
RO-OC-A-CO-OR (I)
wobei:
- A eine lineare oder verzweigte C4-Alkylkette ist und
- R ein Alkyl, ein Cycloalkyl oder ein Aryl ist,
und **dadurch gekennzeichnet, dass** der Weichmacher eine Mischung aus Folgendem ist:
- 70 bis 95 Gew.-% Methylglutarsäurediester,
- 5 bis 30 Gew.-% Ethylbernsteinsäurediester und
- 0 bis 10 Gew.-% Adipinsäurediester,
oder dadurch, dass er eine Mischung aus Folgendem ist:
- 95 bis 100 Gew.-% Methylglutarsäurediester und
- 0 bis 5 Gew.-% Ethylbernsteinsäurediester.

2. Zusammensetzung nach Anspruch 1, **dadurch gekennzeichnet, dass** R ein substituiertes oder unsubstituiertes Cycloalkyl mit 5 bis 6 Kohlenstoffatomen, das aus Cyclopentyl oder Cyclohexyl ausgewählt ist, oder ein Aryl ist, das aus Benzyl, Phenyl und deren Isomeren ausgewählt ist.

3. Zusammensetzung nach Anspruch 1, **dadurch gekennzeichnet, dass** R ein verzweigtes oder unverzweigtes Alkyl mit 4 bis 10 Kohlenstoffatome ist, das insbesondere aus n-Butyl, t-Butyl, n-Pentyl, n-Hexyl, n-Heptyl, n-Octyl, Ethylhexyl, iso-Octyl, n-Nonyl, iso-Nonyl, n-Decyl, iso-Decyl ausgewählt ist.

4. Zusammensetzung nach einem der vorhergehenden Ansprüche, **dadurch gekennzeichnet, dass** sie weiterhin eine oder mehrere Verbindungen umfasst, die aus Stabilisatoren, Füllstoffen, Pigmenten, Bioziden, Ruß, Haftungsvermittlern, Viskositätsverminderern, Thixotropiermitteln, Verdickungsmitteln, Treibmitteln, Dispergiermitteln oder anderen Additiven ausgewählt sind.

5. Zusammensetzung nach einem der vorhergehenden Ansprüche, **dadurch gekennzeichnet, dass** der Weichmacher weiterhin eine oder mehrere Verbindungen umfasst, die aus Phthalat, Adipat, Benzoat, Triglycerid oder anderen Polymeren ausgewählt sind.

6. Verwendung der Zusammensetzung nach einem der Ansprüche 1 bis 5 zur Herstellung von Artikeln, die aus Spielzeug, Filmen, Folien, Schläuchen, Beschichtungen, Kabeln, Bodenplatten, insbesondere auf Vinylbasis, Kunstledern, Klebstoffen und Tinten ausgewählt sind.

7. Verwendung der Zusammensetzung nach einem der Ansprüche 1 bis 5 zur Herstellung von Artikeln durch Trockenmisch- oder Extrusionsverfahren.

8. Verwendung der Zusammensetzung nach einem der Ansprüche 1 bis 5 zur Herstellung von Plastisolen vom Pasten-, Film- oder Schaumstofftyp.

## Claims

1. Plasticizing composition comprising a resin of vinyl chloride type and a plasticizing agent which comprises at least one diester of general formula (I):
RO-OC-A-CO-OR (I)
in which:
- A is a linear or branched C₄ aliphatic chain, and
- R is an alkyl, a cycloalkyl or an aryl,
and **characterized in that** the plasticizing agent is a mixture of:
- from 70% to 95% by weight of methylglutaric acid diester,
- from 5% to 30% by weight of ethylsuccinic acid diester, and
- from 0% to 10% by weight of adipic acid diester,
or is a mixture of:
- from 95% to 100% by weight of methylglutaric acid diester, and
- from 0% to 5% by weight of ethylsuccinic acid diester.

2. Composition according to Claim 1, **characterized in that** R is a substituted or unsubstituted cycloalkyl of 5 to 6 carbon atoms chosen from cyclopentyl or cyclohexyl, or an aryl chosen from benzyl, phenyl, and their isomers.

3. Composition according to Claim 1, **characterized in that** R is a branched or unbranched alkyl having from 4 to 10 carbon atoms chosen in particular from n-butyl, t-butyl, n-pentyl, n-hexyl, n-heptyl, n-octyl, ethylhexyl, isooctyl, n-nonyl, isononyl, n-decyl or isodecyl.

4. Composition according to any one of the preceding claims, **characterized in that** it additionally comprises one or more compounds chosen from stabilizers, fillers, pigments, biocides, carbon black, adhesion promoters, viscosity reducers, thixotropic agents, thickening agents, blowing agents, dispersants or other additives.

5. Composition according to any one of the preceding claims, **characterized in that** the plasticizing agent additionally comprises one or more compounds chosen from phthalate, adipate, benzoate, triglyceride or other polymers.

6. Use of the composition according to any one of Claims 1 to 5, for preparing articles selected from toys, films, sheets, flexible pipes, coatings, cables, floor tiles, in particular vinyl-based ones, synthetic leather, adhesives and inks.

7. Use of the composition according to any one of Claims 1 to 5, for the preparation of articles by dry blending or extrusion processes.

8. Use of the composition according to any one of Claims 1 to 5, for the preparation of plastisols of paste, film or foam type.
